# EUROPEAN PATENT APPLICATION

(11) **EP 4 721 642 A1**
(43) Date of publication of application: **08.04.2026**
(21) Application number: 24815383.5
(22) Date of filing: 23.05.2024
(51) Int. Cl.: A61B 3/028, A61B 3/10

(54) **OPHTHALMIC DEVICE**

(30) Priority: 02.06.2023 JP 2023091298
(71) Applicant: Nidek Co., Ltd., Aichi 443-0038 (JP)
(72) Inventor: TACHIBANA Sasagu, Gamagori-shi Aichi 443-0038 (JP); KITAGAWA Masahiro, Gamagori-shi Aichi 443-0038 (JP); HIRAYAMA Yukito, Gamagori-shi Aichi 443-0038 (JP)
(74) Representative: Hoefer & Partner Patentanwälte mbB
(86) International application number: PCT/JP2024/019122
(87) International publication number: WO 2024/247899

(57) **Abstract**

Provided is an ophthalmic device comprising: a visual target presentation optical system that projects visual target optical flux toward an eye to be examined in order to present a visual target to the eye to be examined; and an alignment index presentation optical system that projects an alignment index toward the fundus of the eye to be examined and presents alignment index optical flux to be observed by the subject in order to present the alignment index to the eye to be examined, wherein the visual target optical flux of the visual target presentation optical system passes through a first region on the pupil plane of the eye to be examined, the alignment index optical flux of the alignment index presentation optical system passes through a second region different from the first region on the pupil plane of the eye to be examined, and the second region includes at least a region outside the first region.

## Description

### TECHNICAL FIELD

The present disclosure relates to an ophthalmologic device for examining a subject eye.

### BACKGROUND ART

As an ophthalmologic device for examining a subject eye, for example, a subjective optometry device that subjectively measures optical characteristics of the subject eye is known. As another ophthalmologic device, an eye refractive power measurement apparatus or the like that objectively measures optical characteristics of a subject eye is known.

In these ophthalmologic devices, the relative positional relation between the subject eye and the ophthalmologic device is adjusted (that is, alignment) by detecting the subject eye. For example, in Patent Literature 1, alignment is automatically adjusted by detecting a corneal apex position using an alignment bright spot projected onto a subject eye and moving a measurement unit based on a deviation between the corneal apex position and an alignment reference position.

### CITATION LIST

### PATENT LITERATURE

Patent Literature 1: JP2018-186930A

### SUMMARY OF INVENTION

In recent years, a subject eye may be examined in a state in which the subject wears glasses, demo lenses, or the like. In this case, in the alignment of the subject eye with a device in the related art, the reflected light of the lens may be detected together with the alignment bright spot projected onto the subject eye, and it may not be possible to correctly perform the alignment.

In view of the above problems, a technical object of the present disclosure is to provide an ophthalmologic device capable of easily performing alignment of a subject eye.

In order to solve the above problems, the present disclosure has the following configuration.
(1) An ophthalmologic device according to a first aspect of the present disclosure including: a visual target presenting optical system configured to project a visual target light flux toward a subject eye to present a visual target to the subject eye; and an alignment index presenting optical system configured to project an alignment index toward a fundus of the subject eye to present an alignment index to the subject eye and to present an alignment index light flux to be observed by a subject, in which the visual target light flux of the visual target presenting optical system passes through a first region on a pupil plane of the subject eye, and the alignment index light flux of the alignment index presenting optical system passes through a second region on the pupil plane of the subject eye, the second region being different from the first region, and the second region includes at least a region outside the first region.

### BRIEF DESCRIPTION OF DRAWINGS

[FIG. 1] FIG. 1 is an external view of an ophthalmologic device.
[FIG. 2] FIG. 2 is a diagram illustrating a left eye measurement unit.
[FIG. 3] FIG. 3 is a light beam diagram of an alignment index presenting optical system.
[FIG. 4] FIG. 4 is a diagram illustrating a positional relation between an alignment index light flux and a visual target light flux.
[FIG. 5] FIG. 5 is a schematic configuration diagram of the inside of the ophthalmologic device as viewed from the front direction.
[FIG. 6] FIG. 6 is a schematic configuration diagram of the inside of the ophthalmologic device as viewed from the side direction.
[FIG. 7] FIG. 7 is a schematic configuration diagram of the inside of the ophthalmologic device as viewed from above.
[FIG. 8] FIG. 8 is a diagram illustrating a control system of the ophthalmologic device.
[FIG. 9A] FIG. 9A illustrates a state in which a subject eye E is appropriately aligned.
[FIG. 9B] FIG. 9B illustrates a state in which the alignment of the subject eye E is slightly deviated.
[FIG. 9C] FIG. 9C illustrates a state in which the alignment of the subject eye E is greatly deviated.
[FIG. 10A] FIG. 10A illustrates a state in which the subject eye E is appropriately aligned.
[FIG. 10B] FIG. 10B illustrates a state in which the alignment of the subject eye E is slightly deviated.
[FIG. 10C] FIG. 10C illustrates a state in which the alignment of the subject eye E is greatly deviated.
[FIG. 11] FIG. 11 is an example in which a second region of the alignment index light flux completely overlaps a first region of the visual target light flux.
[FIG. 12] FIG. 12 is an example in which the second region of the alignment index light flux does not overlap the first region of the visual target light flux.
[FIG. 13] FIG. 13 is an example in which the second region of the alignment index light flux partially overlaps the first region of the visual target light flux.

### DESCRIPTION OF EMBODIMENTS

### <Overview>

An overview of an ophthalmologic device according to an embodiment of the present disclosure will be described. Note that items classified in <> below may be used independently or in conjunction with each other.

### <Ophthalmologic Device>

The ophthalmologic device according to the present embodiment may be a device configured to examine a subject eye.

For example, the ophthalmologic device may be a device configured to measure the subject eye to examine the subject eye. As an example, the ophthalmologic device may be a subjective optometry device for subjectively measuring the optical characteristic of the subject eye. As an example, the ophthalmologic device may be an objective optometry device for objectively measuring the optical characteristic of the subject eye. For example, the optical characteristic of the subject eye may be the eye refractive power (for example, at least one of the spherical power, the cylindrical power, and the astigmatic axis angle) of the subject eye.

For example, the ophthalmologic device may be a device configured to capture the subject eye to examine the subject eye. As an example, the ophthalmologic device may be an ophthalmologic imaging device configured to capture the anterior section of the subject eye to acquire anterior section image data of the subject eye, the corneal shape of the subject eye, and the like. As an example, the ophthalmologic device may be an ophthalmologic imaging device configured to capture the fundus of the subject eye to acquire fundus front image data of the subject eye, fundus tomographic image data of the subject eye, and the like.

### <Visual Target Presenting Optical System>

The ophthalmologic device in the present embodiment may include a visual target presenting optical system (for example, a visual target presenting optical system 26). For example, the visual target presenting optical system is configured to project a visual target light flux toward the subject eye in order to present a visual target to the subject eye.

For example, the visual target presenting optical system may include a visual target presenting unit configured to present a visual target (for example, at least one of a fixation target, an examination visual target, and the like) to the subject eye. For example, the visual target presenting unit may include a visible light source for presenting the visual target. For example, the visual target presenting unit may be a light source and a visual target plate. For example, the visual target presenting unit may be a light source and a digital micromirror device (DMD). For example, the visual target presenting unit may be a display. As an example, the display may be a liquid crystal display (LCD) or an organic electro luminescence (EL) display. As an example, a light field display configured to reproduce a light beam (for example, a light beam reflected by an object) emitted by an object by emitting light different for each direction from pixel set units may be used as the display.

### <Alignment Index Presenting Optical System>

The ophthalmologic device in the present embodiment may include an alignment index presenting optical system (for example, an alignment index presenting optical system 45). For example, in order to present an alignment index to the subject eye, the alignment index presenting optical system is configured to project an alignment index light flux toward the fundus of the subject eye to present the alignment index to be observed by the subject.

For example, the alignment index presenting optical system may include an alignment index presenting unit configured to present an alignment index to the subject eye. For example, the alignment index presenting unit may include a visible light source for presenting the alignment index. For example, the alignment index presenting unit may be a display (as an example, an LCD, an organic EL, or a light field display). For example, the alignment index presenting optical system may include one or more optical members for guiding the alignment index light flux from the alignment index presenting unit.

For example, the alignment index presenting optical system may include one alignment index presenting unit. For example, in this case, the alignment index light flux may be projected as one light flux from the alignment index presenting unit. For example, in this case, the alignment index light flux may be projected as one light flux from the alignment index presenting unit, and further divided into a plurality of alignment index light fluxes via the light flux dividing member. As an example, the light flux dividing member may be at least one of a lens array and a light shielding mask. As an example, the light flux dividing member may be provided at a pupil conjugate position (a substantially pupil conjugate position).

For example, the alignment index presenting optical system may include a plurality of alignment index presenting units. For example, in this case, the alignment index light flux may be projected from each of the alignment index presenting units. That is, a plurality of alignment index light fluxes may be projected.

For example, the alignment index presenting unit provided in the alignment index presenting optical system and the visual target presenting unit provided in the visual target presenting optical system may be provided as different presenting units. For example, the alignment index presenting unit provided in the alignment index presenting optical system and the visual target presenting unit provided in the visual target presenting optical system may be provided as a common presenting unit. That is, the alignment index presenting unit and the visual target presenting unit may be combined.

For example, the alignment index presenting optical system may be configured to present an alignment index for causing the subject to recognize that at least a deviation (an alignment deviation) between the subject and the ophthalmologic device has occurred. For example, the alignment index presenting optical system may be configured to present the alignment index such that the subject can identify the alignment index according to the alignment deviation between the subject and the ophthalmologic device. For example, the alignment index presenting optical system may be configured to present the alignment index so as to change the appearance of the alignment index identified by the subject. As an example, in this case, the alignment index presenting optical system may be configured to present the alignment index such that whether the subject can observe the alignment index changes depending on the alignment deviation between the subject and the ophthalmologic device. As an example, in this case, the alignment index presenting optical system may be configured to present the alignment index such that at least one state of luminance, saturation, contrast, and the like of the alignment index for identifying the subject changes depending on the alignment deviation between the subject and the ophthalmologic device.

For example, the alignment index presenting optical system may be configured to present an alignment index as a guide index indicating a direction in which the subject moves the subject eye, in addition to causing the subject to recognize that the alignment deviation has occurred. For example, in this case, the alignment index presenting optical system may present the alignment index (for example, the guide index) such that whether the subject can observe the alignment index indicating a specific direction changes depending on the alignment deviation between the subject and the ophthalmologic device.

For example, the alignment index presenting optical system may indicate the direction in which the subject moves the subject eye by the shape of the alignment index (the guide index). For example, in this case, the alignment index may have a shape having a specific directionality. As an example, the alignment index may have a triangular shape, and the direction may be indicated by the vertex of the triangle. As an example, the alignment index may have an arrow shape. Of course, the alignment index may have a shape different from a triangular shape or an arrow shape.

For example, the alignment index presenting optical system may indicate the direction in which the subject moves the subject eye by a symbol included in the alignment index (the guide index). For example, in this case, the alignment index may be a character (as an example, "right") representing a specific direction, or an icon representing a specific direction.

For example, the alignment index presenting optical system may indicate the direction in which the subject moves the subject eye by the color of the alignment index (the guide index). For example, in this case, the alignment index may be implemented such that the appearance of the color changes depending on a specific direction.

For example, the alignment index presenting optical system may allow the subject to recognize the alignment deviation. For example, in this case, the alignment index presenting optical system may project the alignment index light flux from the alignment index presenting unit and present an image (in other words, an alignment index image) of the alignment visual target light flux to the subject.

For example, the alignment index presenting optical system may allow the subject to recognize the movement direction of the subject eye. For example, in this case, the alignment index presenting optical system may project the alignment index light flux from the alignment index presenting unit and further form the alignment index light flux into a predetermined pattern shape via the light flux forming member. As an example, the light flux dividing member may be a light shielding mask or the like, and the opening of the light shielding mask may have a shape having a predetermined directionality. For example, accordingly, the image of the alignment visual target light flux that can be observed by the subject is shown as a shape having a predetermined directionality.

For example, the alignment index presenting optical system may enable the subject to recognize the movement direction of the subject eye, may project the alignment index light flux from the alignment index presenting unit, and may change the color of the alignment index light flux to a predetermined color via a color member. As an example, the color member may be a color filter or the like, and the color of the color filter may be different for each specific direction. For example, in this case, the color of the image of the alignment visual target light flux that can be observed by the subject changes depending on the alignment deviation.

For example, by providing the ophthalmologic device according to the present embodiment with such a configuration, when the subject subjectively recognizes the alignment deviation between the subject eye and the ophthalmologic device, the subject can easily grasp the direction for correcting the deviation. The subject can position the subject eye at the correct alignment position by the subject himself or herself only by moving the face in the direction indicated by the guide index.

### <First Region and Second Region on Pupil Plane of Subject Eye>

In the present embodiment, the visual target presenting optical system may be configured such that the visual target light flux passes through a first region on the pupil plane of the subject eye, and the alignment index presenting optical system may be configured such that the alignment index light flux passes through a second region different from the first region on the pupil plane of the subject eye. That is, the visual target light flux of the visual target presenting optical system may be set to pass through the first region on the pupil plane, and the alignment index light flux of the alignment index presenting optical system may be set to pass through the second region on the pupil plane. In this case, as the configuration in which the alignment index light flux passes through the second region different from the first region on the pupil plane of the subject eye, the second region may include at least the outside of the first region through which the visual target light flux passes. The region outside the first region may be a region adjacent to the first region or a region away from the first region.

For example, the entire region of the first region may be included in the second region on the pupil plane of the subject eye. In other words, the entire region of the first region may overlap the second region, and the second region may be present outside the first region. For example, at least a part region of the first region may be outside the second region. More specifically, the second region may partially overlap the first region, and the second region may present outside the first region. Alternatively, the second region may be present outside the first region without overlapping the first region.

For example, by providing the ophthalmologic device according to the present embodiment with such a configuration, the amount of the alignment index light flux toward the fundus increases or decreases depending on the degree of the alignment deviation between the subject eye and the ophthalmologic device, and thus the appearance of the alignment index image that can be observed by the subject changes. Therefore, the subject can easily recognize the occurrence of the alignment deviation using the appearance of the alignment index image.

For example, on the pupil plane of the subject eye, the first region through which the visual target light flux of the visual target presenting optical system passes may be a pupil center region including the pupil center of the subject eye, and the second region through which the alignment index light flux of the alignment index presenting optical system passes may include at least a region outside the pupil center region. For example, the pupil center region of the subject eye may be a predetermined region with respect to the pupil center position of the subject eye. For example, the region outside the pupil center region of the subject eye may be a region separated from the pupil center position of the subject eye by a predetermined distance in each meridian direction.

For example, by providing the ophthalmologic device according to the present embodiment with such a configuration, the visibility (as an example, the defect or brightness of the alignment index image) of the alignment index image is the same regardless of the direction in which the alignment deviation between the subject eye and the ophthalmologic device occurs. More specifically, from a state in which the optical axis of the visual target presenting optical system and the pupil center position of the subject eye coincide (substantially coincide) with each other, the visibility of the alignment index image is the same regardless of the direction in which the pupil center position deviates from the optical axis. Therefore, the subject can easily move the subject eye to a correct position by moving the face or the like.

For example, on the pupil plane of the subject eye, the second region through which the alignment index light flux of the alignment index presenting optical system passes may be located in the regions in the left direction and the right direction with respect to the pupil center position of the subject eye. For example, the left direction with respect to the pupil center position of the subject eye may be a substantially left direction. For example, the left direction may be the left side with respect to the axis (that is, the Y axis passing through the pupil center position of the subject eye) in the vertical direction passing through the pupil center position of the subject eye. For example, the right direction with respect to the pupil center position of the subject eye may be a substantially right direction. For example, the right direction may be the right side with respect to the axis in the vertical direction passing through the pupil center position of the subject eye.

For example, the second region in the left direction through which the alignment index light flux passes may be a region in a direction of 0 degrees to 45 degrees and a region in a direction of 225 degrees to 360 degrees with the axis (that is, the X axis passing through the pupil center position of the subject eye) in the horizontal direction passing through the pupil center position of the subject eye as the reference (0 degrees). For example, the second region may preferably be a region in a direction of 0 degrees (360 degrees). For example, the second region in the right direction through which the alignment index light flux passes may be a region in a direction of 135 degrees to 225 degrees with respect to the axis in the horizontal direction passing through the pupil center position of the subject eye. For example, the second region may preferably be a region in a direction of 180 degrees. Accordingly, the subject can easily recognize the alignment deviation of the subject eye with respect to the ophthalmologic device in the left direction and the right direction.

For example, on the pupil plane of the subject eye, the second region through which the alignment index light flux of the alignment index presenting optical system passes may be located in the regions in the upper direction and the lower direction with respect to the pupil center position of the subject eye. For example, the upper direction with respect to the pupil center position of the subject eye may be a substantially upper direction. For example, the upper direction may be the upper side with respect to the axis (that is, the X axis passing through the pupil center position of the subject eye) in the horizontal direction passing through the pupil center position of the subject eye. For example, the lower direction with respect to the pupil center position of the subject eye may be a substantially lower direction. For example, the lower direction may be the lower side with respect to the axis in the horizontal direction passing through the pupil center position of the subject eye.

For example, the second region in the upper direction through which the alignment index light flux passes may be a region in a direction of 45 degrees to 135 degrees with respect to the axis in the horizontal direction passing through the pupil center position of the subject eye. For example, the second region may preferably be a region in a direction of 90 degrees.

For example, the second region in the lower direction through which the alignment index light flux passes may be a region in a direction of 225 degrees to 315 degrees with respect to the axis in the horizontal direction passing through the pupil center position of the subject eye. For example, the second region may preferably be a region in a direction of 270 degrees. Accordingly, the subject can easily recognize the alignment deviation of the subject eye with respect to the ophthalmologic device in the upper direction and the lower direction.

Of course, for example, on the pupil plane of the subject eye, the second region through which the alignment index light flux of the alignment index presenting optical system passes may be located in the regions in the left direction, the right direction, the upper direction, and the lower direction with respect to the pupil center position of the subject eye. For example, the second region may be located in each of regions in oblique directions such as a left upper direction, a left lower direction, a right upper direction, and a right lower direction together with the left, right, upper, and lower directions. For example, the second region may be located in regions in all directions of 0 degrees to 360 degrees.

For example, on the pupil plane of the subject eye, the first region through which the visual target light flux of the visual target presenting optical system passes may be a region in the pupil of the subject eye. For example, the second region through which the alignment index light flux of the alignment index presenting optical system passes may be a region in the pupil of the subject eye. For example, the second region may be a region outside the pupil of the subject eye. Of course, for example, the second region may be a region including a partial region in the pupil of the subject eye and a region outside the pupil of the subject eye.

For example, in a state in which there is no alignment deviation of the subject eye, when both the first region and the second region are regions in the pupil, the visual target light flux and the alignment index light flux are incident on the fundus of the subject eye. Therefore, the subject can recognize the image (the visual target image) of the visual target light flux and the image (the alignment index image) of the alignment index light flux. For example, when alignment deviation occurs in the subject eye, the second region outside the first region deviates from the inside of the pupil, so that the alignment index light flux is not incident on the fundus. Therefore, the subject can recognize only the visual target image. For example, in such a configuration, the subject cannot recognize the alignment index image, so that the subject can recognize the occurrence of the alignment deviation by the subject himself or herself.

For example, in a state in which there is no alignment deviation of the subject eye, when the first region is a region in the pupil and the second region is a region outside the pupil, only the visual target light flux is incident on the fundus of the subject eye. Therefore, the subject can recognize the image (the visual target image) of the visual target light flux. For example, when alignment deviation occurs in the subject eye, the second region outside the first region is located in the pupil, so that the alignment index light flux is incident on the fundus. Therefore, the subject can recognize the alignment index image. For example, in such a configuration, the subject can recognize the alignment index image, so that the subject can recognize the occurrence of the alignment deviation by the subject himself or herself.

For example, in the above configuration, the allowable range of the alignment deviation can be set by appropriately forming the second region with respect to the first region on the pupil plane of the subject eye. For example, the closer the second region is to the first region, the severer the allowable range of alignment deviation can be set. That is, the subject can recognize the alignment index image even in a state in which the face of the subject slightly moves. For example, the farther the second region is from the first region, the more roughly the allowable range of the alignment deviation can be set. That is, the subject can recognize the alignment index image in a state in which the face of the subject moves greatly.

### <Embodiment>

An embodiment of the ophthalmologic device according to the present embodiment will be described.

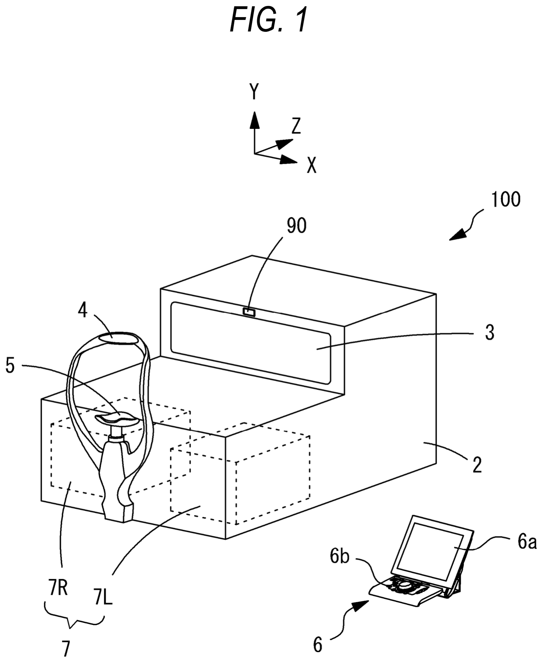

FIG. 1 is an external view of an ophthalmologic device 100. For example, the ophthalmologic device 100 includes a housing 2, a presenting window 3, a forehead rest 4, a jaw support 5, a controller 6, an imaging unit 90, and the like. The housing 2 includes therein a measurement unit 7, a deflection mirror 81, a reflection mirror 84, a concave mirror 85, and the like. The presenting window 3 is used to present a visual target to a subject eye E. The forehead rest 4 and the jaw support 5 are used to maintain a constant distance between the subject eye E and the ophthalmologic device 100. The controller 6 includes a monitor 6a, a switch unit 6b, and the like. The monitor 6a displays various types of information (for example, measurement results of the subject eye). The monitor 6a may be a touch panel that also functions as the switch unit 6b. The switch unit 6b is used to execute various settings (for example, input of a start signal). A signal corresponding to the operation instruction from the controller 6 is output to a control unit 70 by wired communication or wireless communication.

The imaging unit 90 is used to image the face of the subject and adjust the position of the subject eye in the Y direction. The imaging unit 90 includes an imaging optical system (not shown). For example, the imaging optical system may include an imaging element and a lens.

### <Measurement Unit>

The measurement unit 7 includes a left eye measurement unit 7L and a right eye measurement unit 7R. The left eye measurement unit 7L and the right eye measurement unit 7R are formed of the same member. Of course, the left eye measurement unit 7L and the right eye measurement unit 7R may be at least partially formed of different members. The measurement unit 7 includes a pair of left and right subjective measurement units and a pair of left and right objective measurement units (the details will be described later). The visual target light flux and the measurement light flux from the measurement unit 7 are guided to the subject eye E via the presenting window 3.

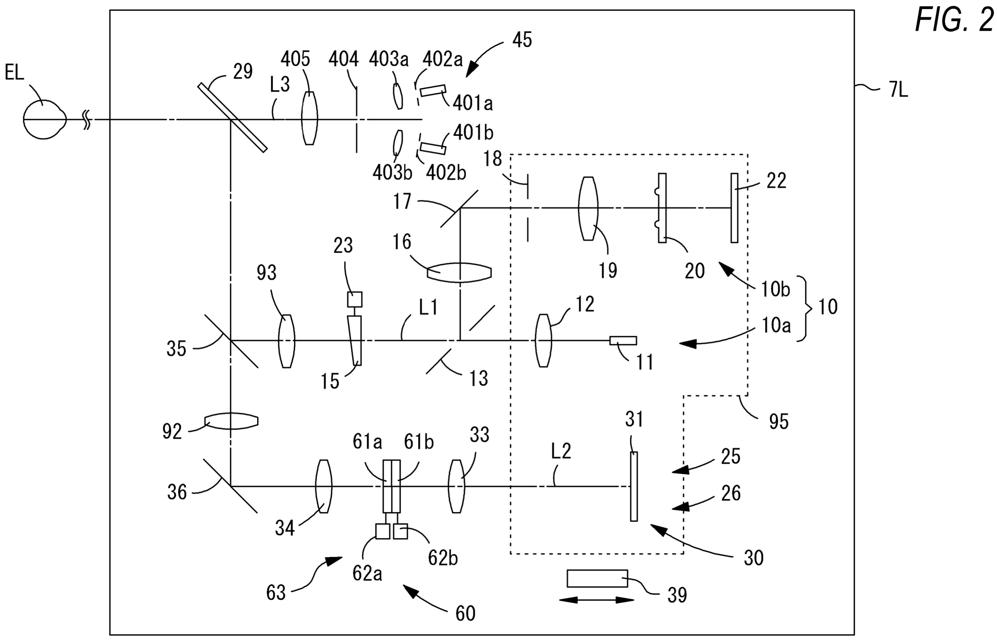

FIG. 2 is a diagram illustrating the left eye measurement unit 7L. The right eye measurement unit 7R has the same configuration as the left eye measurement unit 7L, and thus is omitted. For example, the left eye measurement unit 7L includes the alignment index presenting optical system 45, a subjective measurement optical system 25, and an objective measurement optical system 10. For example, the optical path of the alignment index presenting optical system 45, the optical path of the subjective measurement optical system 25, and the optical path of the objective measurement optical system 10 become a common optical path via a dichroic mirror 29. That is, an optical axis L3 of the alignment index presenting optical system 45, an optical axis L2 of the subjective measurement optical system 25, and an optical axis L1 of the objective measurement optical system 10 are coaxial via the dichroic mirror 29.

### <Alignment Index Presenting Optical System>

The alignment index presenting optical system 45 is configured to project an alignment index light flux, which can be observed by the subject, toward the fundus of the subject eye E in order to present an alignment index to the subject eye E. For example, in the alignment index presenting optical system 45, a light source, an index mask, a relay lens, and the like to be described later are arranged at intervals of 90 degrees on a concentric circle with respect to the optical axis L3. As an example, these optical members are arranged at 0 degrees (the left direction), 90 degrees (the upper direction), 180 degrees (the right direction), and 270 degrees (the lower direction) on the concentric circle with respect to the optical axis L3. That is, for example, a plurality of these optical members are arranged symmetrically with respect to a vertical plane passing through the optical axis L3.

In FIG. 2, the optical members of the alignment index presenting optical system 45 are illustrated only at 0 degrees (the left direction) and 180 degrees (the right direction). Since the configurations of 90 degrees (the upper direction) and 270 degrees (the lower direction) are the same, the description thereof will be omitted. For example, the alignment index presenting optical system 45 includes light sources 401a and 401b, index masks 402a and 402b, relay lenses 403a and 403b, a light receiving diaphragm 404, an objective lens 405, and the dichroic mirror 29.

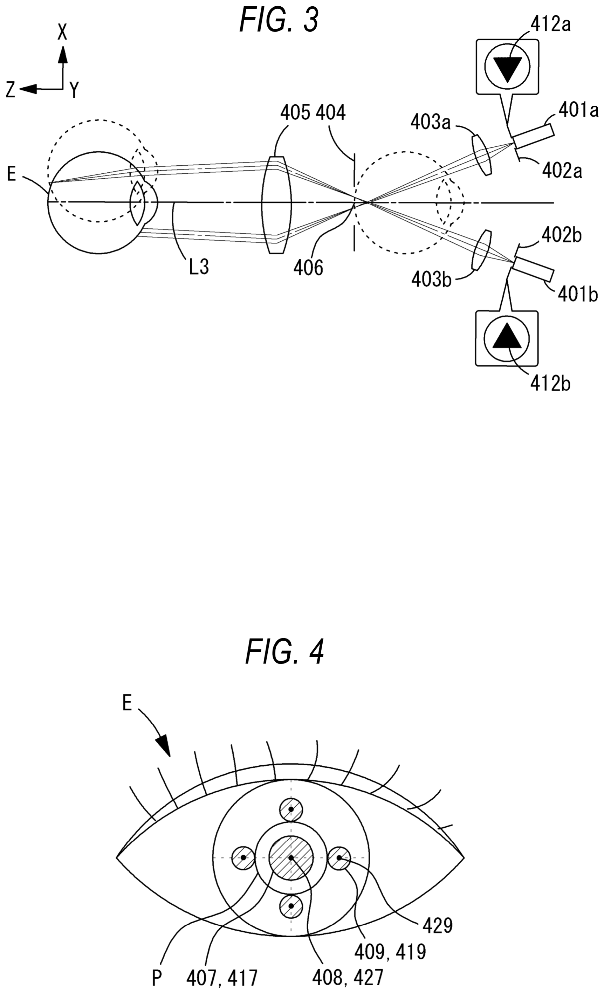

The light sources 401a and 401b is configured to emit alignment index light fluxes. The index masks 402a and 402b is configured to form the alignment index light flux into a predetermined index pattern. The index masks 402a and 402b are arranged at fundus conjugate positions (substantially fundus conjugate positions) of the subject eye E. The index masks 402a and 402b have openings 412a and 412b (see FIG. 3). The relay lenses 403a and 403b is configured to focus the alignment index light flux at a fundus conjugate position (a substantially fundus conjugate position) 406 of the subject eye E. The light receiving diaphragm 404 is provided at the fundus conjugate position (the substantially fundus conjugate position) 406 of the subject eye E, and is configured to prevent stray light generated by diffusion or reflection of the alignment index light flux from being incident on the fundus of the subject eye.

FIG. 3 is a light beam diagram of the alignment index presenting optical system 45. For example, the alignment index light flux from the light source 401a is focused on the fundus conjugate position (the substantially fundus conjugate position) 406 (that is, the position of the light receiving diaphragm 404) via the index mask 402a and the relay lens 403a, and further reaches the subject eye E via the objective lens 405. Similarly, the alignment index light flux from the light source 401b reaches the subject eye E via the optical members.

For example, the alignment index light flux reaches the subject eye E, and the alignment index light flux forms an image on the fundus due to the alignment deviation between the subject eye E and the measurement unit 7, so that the subject can observe an image (in other words, an index pattern image) of the alignment index light flux. The details of the alignment deviation between the subject eye E and the measurement unit 7 and the appearance of the visual target pattern image will be described later.

Here, in the index masks 402a (402b), the shape of the openings 412a (412b) may be any shape that allows the subject to recognize the alignment deviation. For example, the shape of the openings 412a (412b) may be a shape (for example, a perfect circle shape or a regular square shape) having no specific directionality. For example, in this case, when the index pattern image becomes visible, the subject can recognize that the alignment deviation occurs. The shape of the openings 412a (412b) may be any shape that allows the subject to recognize the direction in which the subject eye is moved. For example, the shape of the openings 412a (412b) may be a shape (for example, a triangular shape or an arrow shape) having a specific directionality. For example, in this case, when the index pattern image becomes visible, the subject can recognize that the alignment deviation occurs, and further, the subject can recognize the direction in which the subject eye is moved in order to correct the alignment deviation by himself or herself.

In the present embodiment, a case is exemplified in which the shape of the openings 412a (412b) of the index masks 402a (402b) is a triangular shape. That is, in the present embodiment, the movement of the subject eye in the direction indicated by the triangular shape is guided by the index pattern image.

### <Subjective Measurement Optical System>

The subjective measurement optical system 25 is used as a part of a configuration of a subjective measurement unit configured to subjectively measure the optical characteristic of the subject eye E. In the present embodiment, the eye refractive power of the subject eye E is measured as the optical characteristics of the subject eye E. For example, the subjective measurement optical system 25 includes the visual target presenting optical system 26, a light projecting optical system 30, and a correction optical system 60.

### <Visual Target Presenting Optical System>

The visual target presenting optical system 26 is configured to project a visual target light flux toward the subject eye in order to present a visual target to the subject eye. For example, the visual target presenting optical system 26 includes a display 31. For example, the display 31 is configured to display a visual target (a fixation target, an examination visual target, or the like). The visual target presenting optical system 26 may be used as a part of the configuration of the light projecting optical system 30.

### <Light Projecting Optical System>

The light projecting optical system 30 is configured to project, toward the subject eye E, the visual target light flux emitted from the visual target presenting optical system 26. For example, the light projecting optical system 30 includes the display 31, a light projecting lens 33, a light projecting lens 34, a reflection mirror 36, an objective lens 92, a dichroic mirror 35, and the dichroic mirror 29.

### <Correction Optical System>

The correction optical system 60 is provided in the optical path of the light projecting optical system 30. The correction optical system 60 is configured to change the optical characteristics of the visual target light flux emitted from the display 31. For example, the correction optical system 60 includes an astigmatism correction optical system 63 and a drive mechanism 39. The astigmatism correction optical system 63 is used to correct the cylindrical power and the astigmatic axis angle of the subject eye E. The astigmatism correction optical system 63 is provided between the light projecting lens 33 and the light projecting lens 34. The astigmatism correction optical system 63 includes two positive cylindrical lens 61a and cylindrical lens 61b that have the same focal distance. The cylindrical lens 61a and the cylindrical lens 61b are rotated independently around the optical axis L2 by the driving of a rotation mechanism 62a and a rotation mechanism 62b.

In the present embodiment, a configuration in which the cylindrical lens 61a and the cylindrical lens 61b are used as the astigmatism correction optical system 63 has been described as an example, and the present invention is not limited thereto. The astigmatism correction optical system 63 may correct the cylindrical power, the astigmatic axis angle, and the like. As an example, a correction lens may be inserted into and removed from the optical path of the light projecting optical system 30.

The light source 11 and the relay lens 12 that are provided in a projection optical system 10a, the light receiving diaphragm 18, a collimator lens 19, a ring lens 20, and an imaging element 22 that are provided in a light receiving optical system 10b, and the display 31 provided in the light projecting optical system 30 are integrally movable in the optical axis direction by the drive mechanism 39. That is, the display 31, the light source 11, the relay lens 12, the light receiving diaphragm 18, the collimator lens 19, the ring lens 20, and the imaging element 22 are synchronized as a drive unit 95, and are integrally moved by the drive mechanism 39. The drive mechanism 39 includes a motor and a slide mechanism.

The drive mechanism 39 is configured to move the display 31 in the optical axis L2 direction by moving the drive unit 95 in the optical axis direction. Accordingly, in the objective measurement, fogging can be applied to the subject eye E. In the subjective measurement, it is possible to correct the spherical power of the subject eye E by optically changing the presentation distance of the visual target with respect to the subject eye E. That is, the configuration in which the display 31 is moved in the optical axis L2 direction is used as a spherical power correction optical system that corrects the spherical power of the subject eye E, and the spherical power of the subject eye E is corrected by changing the position of the display 31. The configuration of the spherical power correction optical system may be different from that of the present embodiment. For example, the spherical power may be corrected by arranging a large number of optical elements in the optical path. For example, the spherical power may be corrected by providing the lens in the optical path and moving the lens in the optical axis direction.

The drive mechanism 39 is configured to move the light source 11 and the relay lens 12, and the imaging element 22 from the light receiving diaphragm 18 in the optical axis L1 direction by moving the drive unit 95 in the optical axis direction. Accordingly, the light source 11, the light receiving diaphragm 18, and the imaging element 22 are optically conjugate with the fundus of the subject eye E. Regardless of the movement of the drive unit 95, the hole mirror 13 and the ring lens 20 are conjugate with the pupil of the subject eye E at a constant magnification. Therefore, the fundus reflection light flux obtained by reflecting the measurement light flux of the projection optical system 10a is always incident on the ring lens 20 of the light receiving optical system 10b as a parallel light flux, and a ring-shaped light flux having the same size as the ring lens 20 is imaged by the imaging element 22 in a focused state regardless of the eye refractive power of the subject eye E.

### <Optical Axis and Main Light Beam of Alignment Index Presenting Optical System and Visual Target Presenting Optical System>

In the present embodiment, in a state in which the subject eye E and the measurement unit 7 are appropriately aligned, the optical axis L3 of the alignment index presenting optical system 45 and the optical axis L2 of the visual target presenting optical system 26 (that is, the subjective measurement optical system 25) are provided at the pupil center position (the substantially pupil center position) of the subject eye E. For example, the alignment index presenting optical system 45 is implemented such that the main light beam of the alignment index light flux emitted from the outside of the optical axis L3 by the light sources passes through a position different from the optical axis L3 on the pupil plane of the subject eye E. For example, in the visual target presenting optical system 26, the main light beam of the visual target light flux emitted by the display 31 coincides (substantially coincides) with the optical axis L2. That is, the main light beam of the visual target light flux of the visual target presenting optical system 26 passes through the same position as the optical axis L2 on the pupil plane of the subject eye E.

FIG. 4 is a diagram illustrating a positional relation between the alignment index light flux and the visual target light flux on the pupil plane of the subject eye E. For example, in a state in which the subject eye E and the measurement unit 7 are appropriately aligned, the visual target light flux 407 from the visual target presenting optical system 26 is incident on a pupil P of the subject eye E. For example, the visual target light flux 407 from the visual target presenting optical system 26 has a diameter smaller than the diameter of the pupil P on the pupil plane, and passes through a first region 417 of the pupil P.

For example, in the present embodiment, the first region 417 on the pupil plane of the subject eye E is a pupil center region including the pupil center position of the subject eye E. For example, the first region 417 may be a region with respect to a pupil center position 408. For example, in this case, the center (a main light beam 427 of the visual target light flux 407) of the diameter of the visual target light flux 407 is provided at the pupil center position 408.

On the other hand, for example, in a state in which the subject eye E and the measurement unit 7 are appropriately aligned, an alignment index light flux 409 from the alignment index presenting optical system 45 reaches the subject eye E. For example, since the light sources of the alignment index presenting optical system 45 are located in the upper, lower, left, and right directions with respect to the optical axis L3, the alignment index light flux 409 reaches the subject eye E from each of the four light sources. For example, the alignment index light flux 409 from the alignment index presenting optical system 45 is provided in a second region 419 different from the first region 417 of the pupil P. For example, the second region 419 may be formed at least in a region outside the first region 417. For example, the alignment index light flux 409 may be designed to be located outside the pupil P (as an example, at the iris).

For example, in the present embodiment, the first region 417 on the pupil plane of the subject eye E is the pupil center region with respect to the pupil center position as described above. Therefore, the second region 419 is a region including at least a region outside the pupil center region. As an example, the second region 419 may be a region separated from the pupil center position 408 of the subject eye E by a predetermined distance in any meridian direction. For example, in this case, the center (the main light beam 429 of the alignment index light flux 409) of the diameter of the alignment index light flux 409 is provided at a position different from the pupil center position 408.

Therefore, on the pupil plane of the subject eye E, the main light beam 427 of the visual target light flux 407 of the visual target presenting optical system 26 and the main light beam 429 of the alignment index light flux 409 of the alignment index presenting optical system 45 are provided at different positions. In other words, on the pupil plane of the subject eye E, the main light beam 427 of the visual target light flux 407 and the main light beam 429 of the alignment index light flux 409 are provided at positions where the main light beam 427 and the main light beam 429 do not intersect each other.

### <Objective Measurement Optical System>

The objective measurement optical system 10 is used as a part of a configuration of an objective measurement unit that objectively measures the optical characteristics of the subject eye. In the present embodiment, the eye refractive power of the subject eye E is measured as the optical characteristics of the subject eye E. For example, the objective measurement optical system 10 includes the projection optical system 10a and the light receiving optical system 10b.

The projection optical system 10a projects a spot-shaped measurement visual target onto the fundus of the subject eye E via the pupil center portion of the subject eye E. For example, the projection optical system 10a includes the light source 11, the relay lens 12, the hole mirror 13, a prism 15, an objective lens 93, the dichroic mirror 35, and the dichroic mirror 29. The light source 11 is configured to emit a measurement light flux. The light source 11 is conjugate with the fundus of the subject eye E. The hole of the hole mirror 13 is conjugate with the pupil of the subject eye E. The prism 15 is a light flux deflection member. The prism 15 is provided at a position deviated from a position conjugate with the pupil of the subject eye E, and decenters the measurement light flux passing through the prism 15 with respect to the optical axis L1. The prism 15 is rotationally driven about the optical axis L1 by a drive unit (a motor) 23. The dichroic mirror 35 uses the optical path of the objective measurement optical system 10 and the optical path of the subjective measurement optical system 25 as a common optical path. That is, the optical axis L1 of the objective measurement optical system 10 and the optical axis L2 of the subjective measurement optical system 25 are coaxial. The dichroic mirror 29 is an optical path branching member. The dichroic mirror 29 reflects the measurement light flux from the projection optical system 10a and the visual target light flux from a light projecting optical system 30 (to be described later) and guides the measurement light flux and the visual target light flux to the subject eye E.

The light receiving optical system 10b extracts the fundus reflection light flux reflected by the fundus of the subject eye E in a ring shape via the pupil peripheral portion of the subject eye E. For example, the light receiving optical system 10b includes the dichroic mirror 29, the dichroic mirror 35, the objective lens 93, the prism 15, the hole mirror 13, a relay lens 16, a mirror 17, the light receiving diaphragm 18, the collimator lens 19, the ring lens 20, and the imaging element 22. The ring lens 20 includes a lens portion formed in a ring shape and a light shielding portion in which a light shielding coating is applied to a region other than the lens portion. The ring lens 20 has an optically conjugate positional relation with the pupil of the subject eye E. The light receiving diaphragm 18 and the imaging element 22 have a conjugate relation with the fundus of the subject eye E. An output from the imaging element 22 is input to the control unit 70.

In the present embodiment, the prism 15 is provided on the common optical axis of the projection optical system 10a and the light receiving optical system 10b. For example, since the measurement light flux from the projection optical system 10a passes through the prism 15 and is incident on the subject eye E, and the fundus reflection light flux reflected by the fundus of the subject eye E passes through the same prism 15, the subsequent optical systems perform reverse scanning as if the projection light flux and the fundus reflection light flux (the received light flux) on the pupil are not eccentric.

### <Internal Configuration of Ophthalmologic Device>

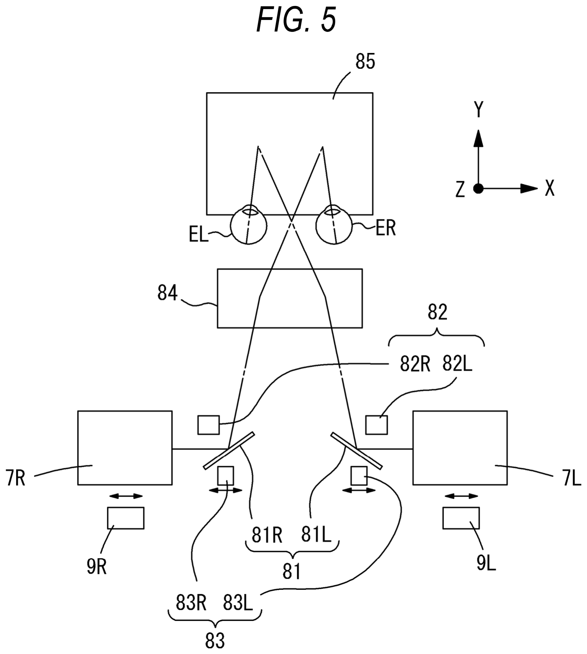

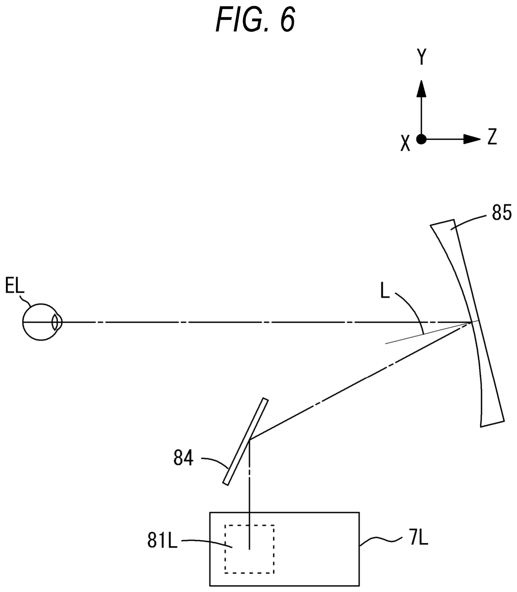

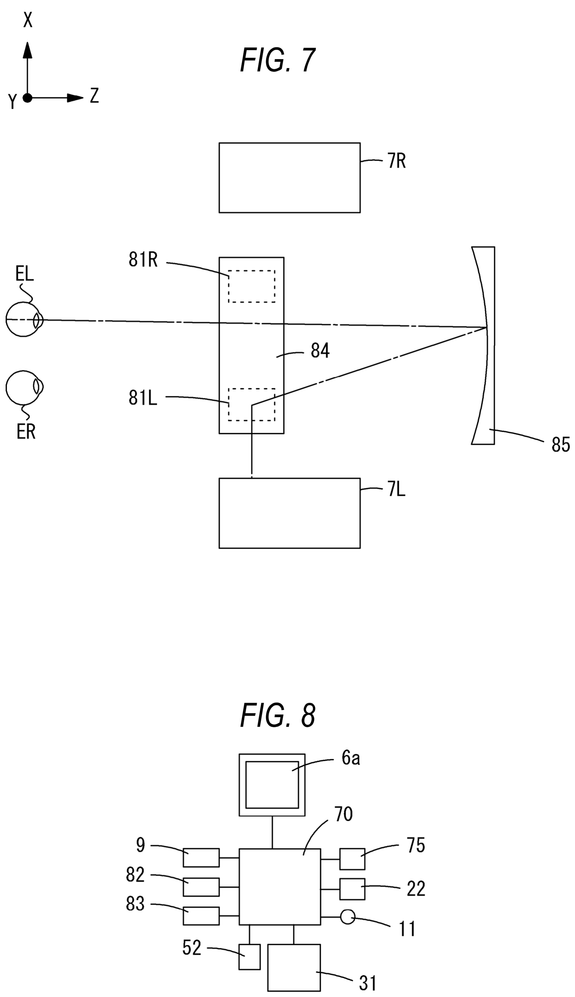

The internal configuration of the ophthalmologic device 100 will be described. FIG. 5 is a schematic diagram of the inside of the ophthalmologic device 100 as viewed from the front direction. FIG. 6 is a schematic diagram of the inside of the ophthalmologic device 100 as viewed from the side direction. FIG. 7 is a schematic diagram of the inside of the ophthalmologic device 100 as viewed from above. In FIGS. 6 and 7, only the optical axis of the left eye measurement unit 7L is shown for convenience of description.

The ophthalmologic device 100 includes an objective measurement unit. For example, the objective measurement unit includes the measurement unit 7, the deflection mirror 81, the reflection mirror 84, and the concave mirror 85. The ophthalmologic device 100 includes a subjective measurement unit. For example, the subjective measurement unit includes the measurement unit 7, the deflection mirror 81, the reflection mirror 84, and the concave mirror 85. The objective measurement unit and the subjective measurement unit are not limited to this configuration. For example, the reflection mirror 84 may be omitted. In this case, the light flux from the measurement unit 7 may be emitted from the oblique direction with respect to the optical axis L of the concave mirror 85 after passing through the deflection mirror 81. For example, a half mirror may be provided. In this case, the light flux from the measurement unit 7 may be emitted from the oblique direction with respect to the optical axis L of the concave mirror 85 via the half mirror.

For example, the deflection mirror 81 includes a left eye deflection mirror 81L and a right eye deflection mirror 81R that are paired and that are respectively provided on the left and right sides. For example, the deflection mirror 81 is provided between the correction optical system 60 and the subject eye E. That is, the correction optical system 60 in the present embodiment includes a left eye correction optical system and a right eye correction optical system that are paired and that are provided on the left and right sides, the left eye deflection mirror 81L is provided between the left eye correction optical system and a left eye EL, and the right eye deflection mirror 81R is provided between the right eye correction optical system and a right eye ER. For example, the deflection mirror 81 is preferably provided at the pupil conjugate position.

For example, the left eye deflection mirror 81L reflects the light flux projected from the left eye measurement unit 7L and guides the light flux to the left eye EL. For example, the left eye deflection mirror 81L reflects the fundus reflection light flux from the left eye EL and guides the fundus reflection light flux to the left eye measurement unit 7L. For example, the right eye deflection mirror 81R reflects the light flux projected from the right eye measurement unit 7R and guides the light flux to the right eye ER. For example, the right eye deflection mirror 81R reflects the fundus reflection light flux from the right eye ER and guides the fundus reflection light flux to the right eye measurement unit 7R.

For example, the deflection mirror 81 is rotationally moved by the drive unit 82. For example, the formation position of the image of the visual target light flux can be optically corrected by deflecting the apparent light flux for forming the image of the visual target light flux in front of the subject eye by the rotational movement of the deflection mirror 81. For example, the drive unit 82 includes a motor or the like. For example, the drive unit 82 rotates the deflection mirror 81 with respect to a rotation axis in the horizontal direction (the X direction) and a rotation axis in the vertical direction (the Y direction). That is, the drive unit 82 rotates the deflection mirror 81 in the XY directions. The deflection mirror 81 may be rotated in one of the horizontal direction and the vertical direction. For example, the drive unit 82 includes a drive unit 82L that drives the left eye deflection mirror 81L and a drive unit 82R that drives the right eye deflection mirror 81R.

In the present embodiment, the configuration in which the deflection mirror 81 is used as the deflection member that reflects and guides the light flux projected from the measurement unit 7 to the subject eye E has been described as an example, and the present invention is not limited thereto. The deflection member may be a prism, a lens, or the like as long as the deflection member can reflect and guide the light flux projected from the measurement unit 7 to the subject eye E.

For example, a plurality of deflection mirrors 81 may be provided in each of the left eye optical path and the right eye optical path. For example, two deflection mirrors may be provided in each of the left eye optical path and the right eye optical path (for example, two deflection mirrors may be provided in the left eye optical path). In this case, one deflection mirror may be rotated in the X direction, and the other deflection mirror may be rotated in the Y direction. For example, by rotationally moving the deflection mirror 81, the apparent light flux for forming the image of the visual target light flux in front of the subject eye can be deflected, and the formation position of the image of the visual target light flux can be optically corrected.

For example, the concave mirror 85 guides the visual target light flux passing through the correction optical system 60 to the subject eye E, and forms an image of the visual target light flux passing through the correction optical system 60 in front of the subject eye E. For example, the concave mirror 85 is shared by the left eye measurement unit 7L and the right eye measurement unit 7R. For example, the concave mirror 85 is shared by the left eye optical path including the left eye correction optical system and the right eye optical path including the right eye correction optical system. That is, the concave mirror 85 is provided at a position through which both the left eye optical path including the left eye correction optical system and the right eye optical path including the right eye correction optical system pass. Of course, the concave mirror 85 may not be shared by the left eye optical path and the right eye optical path. That is, a concave mirror may be provided in each of the left eye optical path including the left eye correction optical system and the right eye optical path including the right eye correction optical system. For example, the concave mirror 85 guides the visual target light flux passing through the correction optical system 60 to the subject eye E, and forms an image of the visual target light flux passing through the correction optical system 60 in front of the subject eye E.

The deflection mirror 81 is driven by a drive unit 83 (for example, a motor). For example, the drive unit 83 includes a left drive unit 83L that drives the left eye deflection mirror 81L and a right drive unit 83R that drives the right eye deflection mirror 81R. The deflection mirrors move in the X direction by driving of the drive unit 83. For example, the distance between the left eye deflection mirror 81L and the right eye deflection mirror 81R is changed by moving the left eye deflection mirror 81L and the right eye deflection mirror 81R, and the distance between the left eye optical path and the right eye optical path in the X direction can be changed according to the pupillary distance of the subject eye E.

The measurement unit 7 is driven by a drive unit 9 (for example, a motor). For example, the drive unit 9 includes a left drive unit 9L that drives the left eye measurement unit 7L and a right drive unit 9R that drives the right eye measurement unit 7R. The measurement unit moves in the X direction by driving of the drive unit 9. For example, by moving the left eye measurement unit 7L and the right eye measurement unit 7R, the distance between the measurement units and the deflection mirror 81 is changed, and the presentation position of the visual target light flux from the measurement units in the Z direction is changed. Accordingly, the measurement unit 7 can be adjusted in the Z direction such that the visual target light flux corrected by the correction optical system 60 is guided to the subject eye E and an image of the visual target light flux corrected by the correction optical system 60 is formed on the fundus of the subject eye E.

### <Optical Paths of Objective Measurement Unit and Subjective Measurement Unit>

The optical path of the objective measurement unit will be described by taking the left eye optical path as an example. The right eye optical path has the same configuration as the left eye optical path. The measurement light flux emitted from the light source 11 of the projection optical system 10a reaches the left eye EL via the optical members. For example, the measurement light flux is guided from the left eye measurement unit 7L to the left eye deflection mirror 81L by sequentially passing through the optical members from the relay lens 12 to the dichroic mirror 29. Further, the visual target light flux is reflected by the left eye deflection mirror 81L, and is guided to the left eye EL via the reflection mirror 84 and the concave mirror 85. A spot-shaped point light source image is formed on the fundus of the left eye EL. At this time, the pupil projection image (the projection light flux on the pupil) of the hole in the hole mirror 13 is eccentrically rotated at high speed by the prism 15 rotating around the optical axis.

The measurement light flux is reflected and emitted from the fundus of the subject eye E, and is guided to the left eye measurement unit 7L via the concave mirror 85, the reflection mirror 84, and the deflection mirror 81. Further, when the light is reflected by the dichroic mirror 29 and the dichroic mirror 35, condensed by the objective lens 93, and condensed again on the opening of the light receiving diaphragm 18 via the prism 15 rotating at high speed and the optical members from the hole mirror 13 to the mirror 17, an image is formed on the imaging element 22 as a ring-shaped image by the collimator lens 19 and the ring lens 20. The optical characteristics of the subject eye E can be objectively measured by analyzing the ring-shaped image captured by the imaging element 22.

The optical path of the subjective measurement unit will be described using the left eye optical path as an example. The right eye optical path has the same configuration as the left eye optical path. The visual target light flux emitted from the display 31 of the subjective measurement optical system 25 reaches the left eye EL via the optical members. For example, the visual target light flux is guided from the left eye measurement unit 7L to the left eye deflection mirror 81L by sequentially passing through the optical members from the light projecting lens 33 to the dichroic mirror 29. Further, the visual target light flux is reflected by the left eye deflection mirror 81L, and is guided to the left eye EL via the reflection mirror 84 and the concave mirror 85.

Accordingly, an image of the visual target light flux corrected by the correction optical system 60 is formed on the fundus of the left eye EL with reference to the glasses wearing position (for example, approximately 12 mm from the corneal apex position) of the left eye EL. Therefore, the fact that the spherical power is adjusted (driving of the drive mechanism 39 in the present embodiment) by the correction optical system in front of the eye is equivalent to the fact that the astigmatism correction optical system 63 is provided as if the astigmatism correction optical system 63 is in front of the eye. The subject can collimate the image of the visual target light flux optically formed in front of the eye at a predetermined examination distance in a natural state via the concave mirror 85.

### <Control Unit>

FIG. 8 is a diagram illustrating a control system of the ophthalmologic device 100. The control unit 70 includes a CPU (a processor), a RAM, a ROM, and the like. For example, the CPU is configured to control the members in the ophthalmologic device 100. For example, the RAM is configured to temporarily store various types of information. For example, the ROM stores various programs for controlling the operation of the ophthalmologic device 100, visual targets, and initial values. The control unit 70 may be implemented by a plurality of control units (that is, a plurality of processors).

For example, various members such as the monitor 6a, the light source 11, the imaging element 22, the display 31, the imaging element 52, and a nonvolatile memory 75 (hereinafter, a memory 75) are electrically connected to the control unit 70. For example, the drive unit 9, the drive unit 82, the drive unit 83, and the drive mechanism 39 are electrically connected to the control unit 70. For example, the memory 75 is, for example, a non-transitory storage medium capable of storing storage contents even when a supply of power is cut off. For example, a hard disk drive, a flash ROM, or a USB memory can be used as the memory 75.

### <Alignment Deviation of Subject Eye and Appearance of Index Pattern Image>

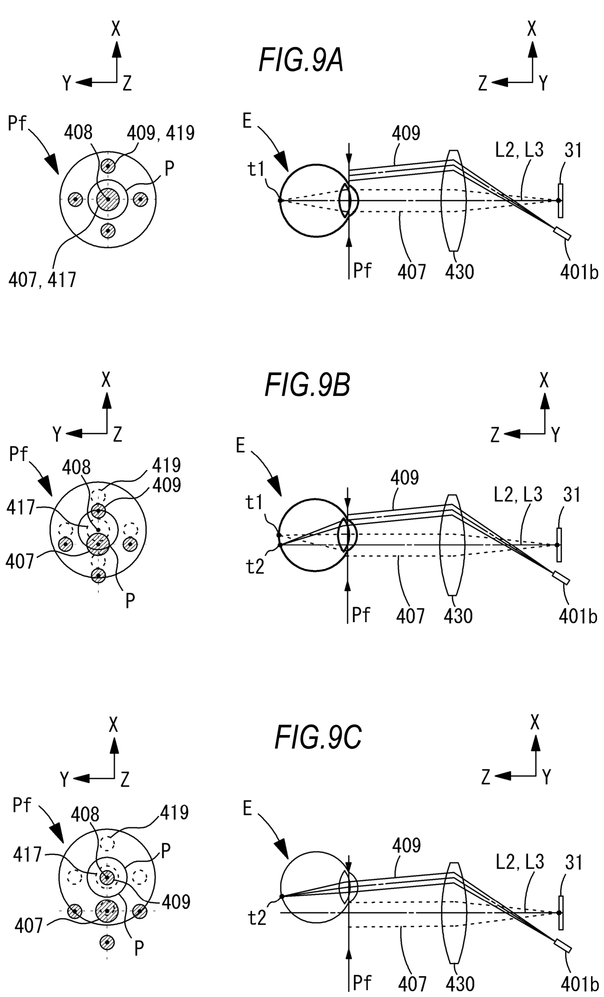

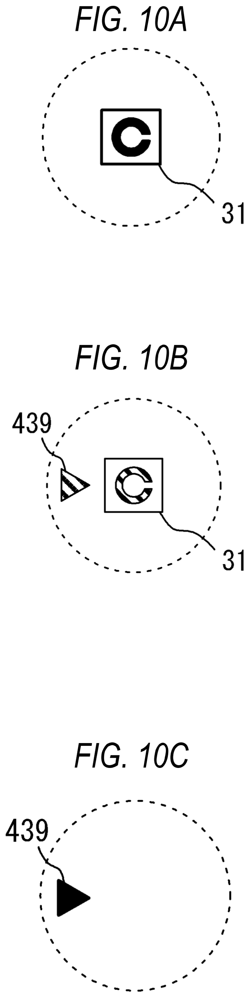

In the present embodiment, the appearance of the index pattern image (the alignment index image) is changed by the alignment index presenting optical system 45 and the visual target presenting optical system 26 described above according to the degree of alignment deviation between the subject eye E and the measurement unit 7. Hereinafter, this will be described in detail with reference to FIGS. 9A to 9C and 10A to 10C.

FIGS. 9A to 9C are diagrams schematically illustrating changes in the positions of the visual target light flux 407 and the alignment index light flux 409 due to alignment deviation of the subject eye E. In FIGS. 9A to 9C, the visual target presenting optical system 26 and the alignment index presenting optical system 45 are simplified, and the lens group (the light projecting lens 33, the light projecting lens 34, the objective lens 92, and the like) constituting the light projecting optical system 30 and the objective lens 405 constituting the alignment index presenting optical system 45 are replaced with one composite lens 430 in the illustration. The alignment index presenting optical system 45 only shows 0 degrees (the left direction). FIGS. 10A to 10C are diagrams schematically illustrating the appearance of a visual target image and an alignment index image due to alignment deviation of the subject eye E. FIGS. 9A and 10A illustrate a state in which the subject eye E is appropriately aligned. FIGS. 9B and 10B illustrate a state in which the alignment of the subject eye E is slightly deviated. FIGS. 9C and 10C illustrate a state in which the alignment of the subject eye E is greatly deviated.

For example, as illustrated in FIG. 9A, a state in which the subject eye E is appropriately aligned is a state in which the pupil center position 408 of the subject eye E, the optical axis L2 of the visual target presenting optical system 26, and the optical axis L3 of the alignment index presenting optical system 45 coincide (substantially coincide) with one another. At this time, the visual target light flux 407 from the visual target presenting optical system 26 is emitted from the display 31 and passes through the first region (the pupil center region) 417 on a pupil plane Pf of the subject eye E via the composite lens 430 and the like. For example, since the visual target light flux 407 has a diameter smaller than the pupil diameter on the pupil plane Pf, all the light fluxes are directed to the fundus without being blocked by the iris or the like and form an image at an imaging position t1 on the fundus. On the other hand, the alignment index light flux 409 from the alignment index presenting optical system 45 is emitted from the light source 401b and reaches a second region 419 (here, a region outside the pupil P) outside the first region 417 on the pupil plane Pf of the subject eye E through the composite lens 430 and the like. However, for example, the alignment index light flux 409 is not imaged on the fundus because all the light fluxes are blocked by the iris.

In a state in which the subject eye E is appropriately aligned, the subject can satisfactorily visually recognize the image of the visual target light flux 407, but cannot visually recognize the image (the alignment index image) of the alignment index light flux 409. For example, as illustrated in FIG. 10A, the Landolt ring visual target displayed on the display 31 can be satisfactorily observed, but the alignment index image cannot be observed. Therefore, when the alignment index image is not visible in the examination of the subject eye E, the subject can easily determine that the alignment deviation does not occur.

For example, as illustrated in FIG. 9B, a state in which the alignment of the subject eye E is slightly deviated is a state in which the pupil center position 408 of the subject eye E does not coincide with the optical axis L2 of the visual target presenting optical system 26 and the optical axis L3 of the alignment index presenting optical system 45. Here, a state in which the face of the subject moves to the left direction from the state in FIG. 9A and the pupil center position 408 of the subject eye E is shifted to the left direction with respect to the optical axis will be described as an example. At this time, the visual target light flux 407 from the visual target presenting optical system 26 is emitted from the display 31 and passes through a region shifted to the right side with respect to the first region (the pupil center region) 417 on the pupil plane Pf of the subject eye E after passing through the composite lens 430 and the like. For example, the visual target light flux 407 passes through a partial region of the first region (the pupil center region) 417 and a region in the pupil P adjacent to the first region 417. Therefore, for example, a part light flux of the visual target light flux 407 is blocked by the iris or the like, and the remaining light flux forms an image at the imaging position t1 toward the fundus. On the other hand, the alignment index light flux 409 from the alignment index presenting optical system 45 is emitted from the light source 401b and passes through a region shifted to the right side with respect to the second region 419 on the pupil plane Pf of the subject eye E via the composite lens 430 and the like. For example, the alignment index light flux 409 passes through a region shifted to the right side with respect to the second region 419 on the pupil plane Pf of the subject eye E. For example, the alignment index light flux 409 passes through a region in the pupil P adjacent to the first region 417. Therefore, for example, a part of the alignment index light flux 409 is blocked by the iris or the like, and the remaining light flux forms an image at the imaging position t2 toward the fundus.

The subject can recognize an image of the visual target light flux 407 and an image (an alignment index image 439) of the alignment index light flux 409 in a state in which the alignment of the subject eye E is slightly deviated. For example, as illustrated in FIG. 10B, both the Landolt ring visual target displayed on the display 31 and the alignment index image 439 appearing around the Landolt ring visual target can be observed. Therefore, when the alignment index image 439 is seen in the examination of the subject eye E, the subject can easily determine that the face moves and alignment deviation occurs.

For example, as illustrated in FIG. 9C, even in a state in which the alignment of the subject eye E is greatly deviated, the pupil center position 408 of the subject eye E does not coincide with the optical axis L2 of the visual target presenting optical system 26 and the optical axis L3 of the alignment index presenting optical system 45. Here, a state in which the face of the subject moves further to the left direction from the state in FIG. 9B and the pupil center position 408 of the subject eye E is shifted to the left direction with respect to the optical axis will be described as an example. At this time, the visual target light flux 407 from the visual target presenting optical system 26 is emitted from the display 31 and reaches a region largely shifted to the right side with respect to the first region (the pupil center region) 417 on the pupil plane Pf of the subject eye E after passing through the composite lens 430 and the like. For example, the visual target light flux 407 reaches a region outside the pupil P. Therefore, for example, all light fluxes of the visual target light flux 407 are blocked by the iris and are not imaged on the fundus. On the other hand, the alignment index light flux 409 from the alignment index presenting optical system 45 is emitted from the light source 401b and passes through a region largely shifted to the right side with respect to the second region 419 on the pupil plane Pf of the subject eye E via the composite lens 430 and the like. For example, the alignment index light flux 409 passes through the first region 417. Therefore, for example, all light fluxes of the alignment index light flux 409 are directed to the fundus without being blocked by the iris or the like, and form an image at the imaging position t2.

The subject cannot visually recognize the image of the visual target light flux 407 in a state in which the alignment of the subject eye E is greatly deviated, and can visually recognize only the image (the alignment index image) of the alignment index light flux 409. For example, as shown in FIG. 10C, the Landolt ring visual target displayed on the display 31 cannot be observed, and only the alignment index image 439 can be observed. Therefore, if only the alignment index image 439 is seen in the examination of the subject eye E, the subject can easily determine that the face moves and the alignment deviation occurs.

In the present embodiment, since the triangular alignment index image 439 is used, the subject can easily grasp the direction in which the subject eye is moved by himself or herself. For example, the subject can place the subject eye E at an appropriate alignment position (in other words, a position where the Landolt ring visual target can be satisfactorily recognized) by moving the face based on the alignment index image 439.

### <Control Operation>

A control operation of the ophthalmologic device 100 having the above configuration will be described.

### <Alignment of Subject Eye>

First, the examiner instructs the subject to cause the face to abut against the forehead rest 4 and the jaw support 5 to observe the presenting window 3. Next, the examiner operates the switch unit 6b to check the alignment state between the subject eye E and the measurement unit 7. For example, in response to a signal received from the switch unit 6b, the control unit 70 turns on the light source 401 of the alignment index presenting optical system 45 and displays the Landolt ring visual target on the display 31.

Here, the examiner asks the subject whether only the Landolt ring visual target can be recognized. For example, when the subject answers that the alignment index image can be recognized, the examiner adjusts the positions of the forehead rest 4 and the jaw support 5 to place the subject eye E at a position where the alignment index image cannot be seen. Alternatively, an instruction to move the face may be issued to the subject, and the subject eye E may be placed at a position where the alignment index image is not visible to the subject. For example, accordingly, the alignment of the subject eye E with respect to the measurement unit 7 in the XY directions is completed.

Since the face of the subject is fixed to the forehead rest 4 and the jaw support 5, the subject eye E is less likely to move in the Z direction. Therefore, the position of the measurement unit 7 in the Z direction inside the ophthalmologic device may be set in advance based on the position at which the subject eye E is roughly placed and the working distance from the subject eye E to the measurement unit 7. For example, in this case, the subject causes the face to abut against the forehead rest 4 and the jaw support 5 to complete the alignment of the subject eye E with respect to the measurement unit 7 in the Z direction.

### <Subjective Examination>

When the examiner checks the alignment state between the subject eye E and the measurement unit 7, the examiner starts subjective measurement on the subject eye E. At this time, when the alignment index image is visible to the subject during the measurement of the subject eye E, the examiner may issue an instruction to move the face such that the alignment index image does not enter the field of view.

The examiner operates the switch unit 6b to select a correction power (for example, at least one of a spherical power, a cylindrical power, and an astigmatic axis angle) for calibrating the subject eye E. For example, the control unit 70 controls at least one of the light projecting optical system 30 and the correction optical system 60 according to the selection signal from the switch unit 6b. For example, the control unit 70 may correct the spherical power of the subject eye E by driving the drive mechanism 39 and moving the display 31 in the optical axis L2 direction. For example, the control unit 70 may correct at least one of the cylindrical power and the astigmatic axis angle of the subject eye E by driving the rotation mechanism 62a and the rotation mechanism 62b and rotating the cylindrical lens 61a and the cylindrical lens 61b around the optical axis L2b. Accordingly, the subject eye E is corrected with a predetermined diopter value (for example, 0 D).

The examiner operates the switch unit 6b to check whether the correction power for calibrating the subject eye E is appropriate while switching the visual acuity value of the Landolt ring visual target to be presented to the subject eye E. For example, the subject is asked about the direction of the Landolt ring visual target. If the subject makes a correct answer, the visual acuity value is switched to a value that is one step higher (that is, a small value), and if the subject makes an incorrect answer, the visual acuity value is switched to a value that is one step lower (that is, a large value). The control unit 70 changes the Landolt ring visual target to be displayed on the display 31 based on the change signal from the switch unit 6b. When the correction power for calibrating the subject eye E is inappropriate, the correction power is changed. Accordingly, the subjective eye refractive power (the subjective value) of the subject eye E is acquired.

For example, even during the subjective measurement of the subject eye E, the alignment index light flux 409 is projected from the light source 401 of the alignment index presenting optical system 45. For example, when the subject moves the face during the measurement, the alignment index image appears around the Landolt ring visual target as described above, and the occurrence of the alignment deviation can be recognized. For example, the subject can move the face in the direction indicated by the alignment index image by the subject himself or herself, so that the alignment deviation can be corrected as needed, and the measurement can be accurately advanced.

The ophthalmologic device according to the present embodiment includes the alignment index presenting optical system 45, so that it is not necessary to provide an optical system for imaging the anterior section of the subject eye E and an optical system for executing alignment using the alignment bright spot included in the anterior section image. Therefore, for example, even when measurement is executed in a state in which the subject wears glasses or the like, there is no possibility that the reflected light flux or the like from the glasses interferes with the detection of the alignment bright spot and alignment becomes difficult. For example, by reducing the number of optical members in the ophthalmologic device, the ophthalmologic device can be downsized.

As described above, for example, an ophthalmologic device according to the present embodiment includes: a visual target presenting optical system configured to project a visual target light flux toward a subject eye to present a visual target to the subject eye; and an alignment index presenting optical system configured to project an alignment index toward a fundus of the subject eye to present an alignment index to the subject eye and present an alignment index light flux to be observed by a subject, in which the visual target light flux of the visual target presenting optical system passes through a first region on a pupil plane of the subject eye, and the alignment index light flux of the alignment index presenting optical system passes through a second region different from the first region on the pupil plane of the subject eye, and in which the second region includes at least a region outside the first region. Accordingly, for example, the amount of the alignment index light flux directed to the fundus increases or decreases depending on the degree of deviation (that is, alignment deviation) in the positional relation between the subject eye and the ophthalmologic device, and thus the appearance of the image of the alignment index light flux that can be observed by the subject changes. Therefore, the subject can easily and subjectively recognize the alignment deviation using the appearance of the image of the alignment index light flux.

For example, in the ophthalmologic device according to the present embodiment, the first region on the pupil plane of the subject eye is a pupil center region including a pupil center of the subject eye, and the second region on the pupil plane of the subject eye includes at least a region outside the pupil center region. For example, in the alignment between the subject eye and the ophthalmologic device, the optical axis of the visual target presenting optical system and the pupil center of the subject eye coincide (substantially coincide) with each other, so that a certain amount of visual target light flux is directed to the fundus, and the subject can appropriately visually recognize the visual target. On the other hand, for example, when the pupil center is shifted with respect to the optical axis of the visual target presenting optical system, the amount of the visual target light flux toward the fundus changes, and thus the subject eye may not be able to appropriately visually recognize the visual target. However, in the ophthalmologic device according to the present embodiment, the alignment index can be recognized according to such a shift of the pupil center with respect to the optical axis. Regardless of the direction in which the pupil center is shifted with respect to the optical axis, the visibility (as an example, the degree of the brightness or defect of the alignment index) of the alignment index is the same. Therefore, the subject can easily move the subject eye to a correct position by moving the face or the like. As a result, the subject eye is accurately examined.

For example, in the ophthalmologic device according to the present embodiment, the second region on the pupil plane of the subject eye is located in each of a left direction and a right direction with respect to a pupil center position of the subject eye. Accordingly, for example, the subject can easily recognize the alignment deviation of the subject eye with respect to the ophthalmologic device in the left direction and the right direction. For example, when the ophthalmologic device includes a forehead rest or a jaw support, the face of the subject is less likely to move in the upper-lower direction but is likely to move in the left-right direction. Therefore, by forming the second region in at least each of the left direction and the right direction with respect to the pupil center position of the subject eye, it is possible to observe the image of the alignment index light flux corresponding to the alignment deviation of the subject eye in the left-right direction and easily recognize the alignment deviation.

For example, in the ophthalmologic device according to the present embodiment, the second region on the pupil plane of the subject eye is located in each of an upper direction and a lower direction with respect to a pupil center position of the subject eye. Accordingly, for example, the subject can easily recognize the alignment deviation of the subject eye with respect to the ophthalmologic device in the upper direction and the lower direction. For example, even when the ophthalmologic device does not include a forehead rest or a jaw support and the face of the subject is likely to move in the upper-lower direction, by forming the second region in at least each of the upper direction and the lower direction with respect to the pupil center position of the subject eye, it is possible to observe the image of the alignment index light flux according to the alignment deviation of the subject eye in the upper-lower direction and easily recognize the alignment deviation. For example, by forming the second region in each of the upper, lower, left, and right directions with respect to the pupil center position of the subject eye, the subject can more accurately grasp the direction of the alignment deviation.

For example, in the ophthalmologic device according to the present embodiment, the alignment index presenting optical system presents, as the alignment index, a guide index capable of recognizing a direction in which the subject moves the subject eye. Accordingly, for example, when the subject subjectively recognizes the alignment deviation between the subject eye and the ophthalmologic device, the subject can easily grasp the direction for correcting the alignment deviation between the subject eye and the ophthalmologic device. The subject can position the subject eye at the correct alignment position by the subject himself or herself only by moving the face in the direction indicated by the guide index.

### <Modifications>

The ophthalmologic device according to the present embodiment may be implemented such that, on the pupil plane Pf of the subject eye E, the second region 419 through which the alignment index light flux 409 of the alignment index presenting optical system 45 passes is located at least outside the first region (the pupil center region) 417 through which the visual target light flux 407 of the visual target presenting optical system 26 passes. The second region 419 may be located in at least the region in each of the left direction and the right direction with respect to the pupil center position 408 of the subject eye E.

For example, in this case, on the pupil plane Pf of the subject eye E, the entire region of the first region 417 through which the visual target light flux 407 passes may be included in the second region 419 through which the alignment index light flux 409 passes. That is, for example, the second region 419 may completely overlap the first region 417. For example, in this case, on the pupil plane Pf of the subject eye E, at least a partial region of the first region 417 through which the visual target light flux 407 passes may be outside the second region 419 through which the alignment index light flux 409 passes. That is, for example, the second region 419 may not overlap the first region 417. For example, the second region 419 may partially overlap the first region 417.

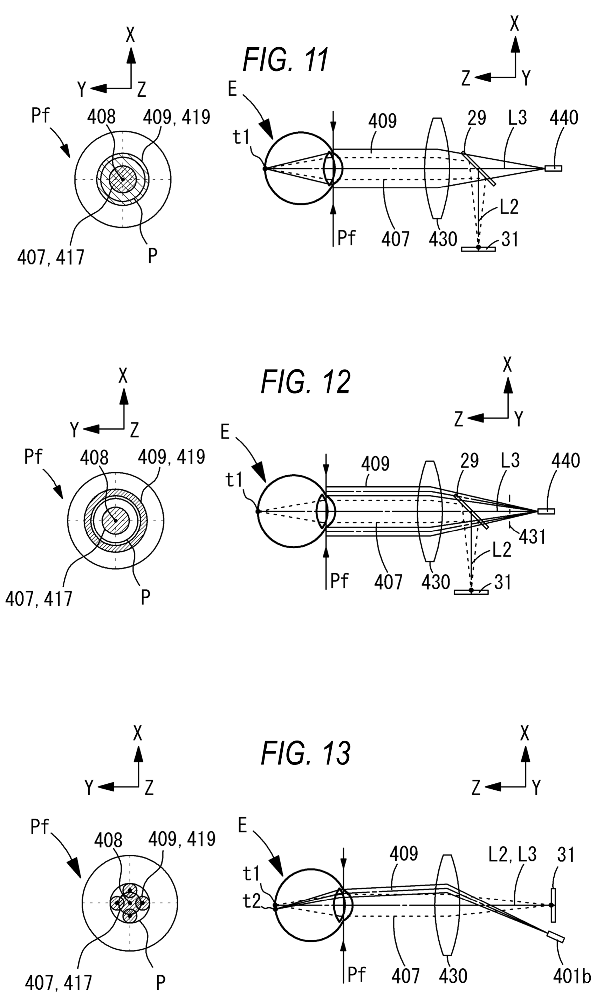

Hereinafter, these configurations will be described in order with reference to FIGS. 11 to 13. In FIGS. 11 to 13, the visual target presenting optical system 26 and the alignment index presenting optical system 45 are simplified as in FIGS. 9A to 9C.

FIG. 11 is an example in which the second region 419 of the alignment index light flux 409 completely overlaps the first region 417 of the visual target light flux 407 on the pupil plane Pf of the subject eye E. For example, in this case, the display 31 may be provided on the optical axis L2 of the visual target presenting optical system 26, and a light source (a light source 440) may be provided on the optical axis L3 of the alignment index presenting optical system 45. Accordingly, the main light beam of the visual target light flux 407 is at the same position as the optical axis L2, and the main light beam of the alignment index light flux 409 is at the same position as the optical axis L3. That is, the main light beam of the visual target light flux 407 and the main light beam of the alignment index light flux 409 are coaxial. For example, the diameter of the alignment index light flux 409 may be larger than the diameter of the visual target light flux 407 and the pupil diameter on the pupil plane Pf.

For example, in the configuration in FIG. 11, the visual target light flux 407 is incident through the first region (the pupil center region) 417 on the pupil plane Pf of the subject eye E. The alignment index light flux 409 is incident through the first region 417 on the pupil plane Pf of the subject eye E and a region in the pupil P adjacent to the first region 417. Therefore, for example, on the fundus of the subject eye E, all light fluxes of the visual target light flux 407 and a part light fluxes of the alignment index light flux 409 are imaged at the imaging position t1. The subject can visually recognize both the Landolt ring visual target and the alignment index in a state in which the subject eye E is appropriately aligned. When alignment deviation occurs in the subject eye E, only the alignment index image is visually recognized depending on the degree of the alignment deviation.

For example, in the configuration in FIG. 11, the alignment index light flux 409 is provided in the directions on the pupil plane Pf of the subject eye E, but is not divided in the directions, and thus it is difficult to recognize the direction of the alignment deviation based on the alignment index image. Therefore, for example, color filters having different colors in the left direction, the right direction, the upper direction, and the lower direction may be arranged at positions different from the fundus conjugate position (the substantially fundus conjugate position) of the subject eye E. As an example, a color filter or the like may be provided at the pupil conjugate position (the substantially pupil conjugate position) of the subject eye E. For example, in this case, since the color of the field of view of the subject changes according to the alignment deviation, the direction can be recognized in which the subject moves the face.

FIG. 12 is an example in which the second region 419 of the alignment index light flux 409 does not overlap the first region 417 of the visual target light flux 407 on the pupil plane Pf of the subject eye E. For example, in this case, a light shielding mask 431 may be further provided in front of the light source 440 in the configuration in FIG. 11. For example, the light shielding mask 431 may have a ring-shaped opening with respect to the optical axis L3. For example, when the alignment index light flux 409 passes through the light shielding mask 431, the alignment index light flux 409 becomes a ring light flux.

For example, in the configuration in FIG. 12, the visual target light flux 407 is incident through the first region (the pupil center region) 417 on the pupil plane Pf of the subject eye E. On the other hand, a part light fluxes of the alignment index light flux 409 near the optical axis L3 are blocked by the light shielding mask 431, and the remaining light fluxes reach a region outside the pupil P. Therefore, for example, on the fundus of the subject eye E, only all light fluxes of the visual target light flux 407 form an image at the imaging position t1. The subject can visually recognize the Landolt ring visual target in a state in which the subject eye E is appropriately aligned. When alignment deviation occurs in the subject eye E, the alignment index image can be visually recognized.

For example, in the configuration in FIG. 12, the alignment index light flux 409 is provided in the directions on the pupil plane Pf of the subject eye E, but is not divided in the directions, and thus it is difficult to recognize the direction of the alignment deviation based on the alignment index image. Therefore, for example, the ring-shaped opening of the light shielding mask 431 may be divided in the left direction, the right direction, the upper direction, and the lower direction. For example, in this case, since an arc-shaped alignment index image corresponding to the alignment deviation appears in the field of view of the subject, the subject can recognize the direction in which the face moves.

For example, in the case of the configuration in FIGS. 11 and 12, since it is not necessary to provide a plurality of light sources as the alignment index presenting optical system 45, the configuration can be simplified.

FIG. 13 is an example in which the second region 419 of the alignment index light flux 409 partially overlaps the first region 417 of the visual target light flux 407 on the pupil plane Pf of the subject eye E. For example, in this case, similarly to FIGS. 9A to 9C, the display 31 may be provided on the optical axis L2 of the visual target presenting optical system 26, and the light source (the light source 401) may be provided outside the optical axis L3 of the alignment index presenting optical system 45.

For example, in the configuration in FIG. 13, the visual target light flux 407 is incident through the first region (the pupil center region) 417 on the pupil plane Pf of the subject eye E. The alignment index light flux 409 is incident through a part of the first region 417 on the pupil plane Pf of the subject eye E and a region in the pupil P adjacent to the first region 417. Therefore, for example, on the fundus of the subject eye E, all light fluxes of the visual target light flux 407 are imaged at the imaging position t1, and a part light fluxes of the alignment index light flux 409 are imaged at the imaging position t2. The subject can visually recognize both the Landolt ring visual target and the alignment index in a state in which the subject eye E is appropriately aligned. When alignment deviation occurs in the subject eye E, only the alignment index image is visually recognized depending on the degree of the alignment deviation.

For example, in the configurations in FIGS. 9A to 9C and FIGS. 11 to 13, the alignment index light flux 409 passes through the directions with respect to the pupil center position 408 on the pupil plane Pf of the subject eye E, so that the second region 419 is provided in the left-right direction and the upper-lower direction of the first region 417. Therefore, even when the face of the subject moves in any direction, the subject can easily subjectively recognize the alignment deviation. Of course, for example, the configurations in FIGS. 9A to 9C and 13 may be designed such that the second region 419 is formed only in the upper-lower direction or the second region 419 is formed only in the left-right direction.

In the ophthalmologic device according to the present embodiment, on the pupil plane Pf of the subject eye E, the second region 419 through which the alignment index light flux 409 of the alignment index presenting optical system 45 passes may be formed in the oblique direction of the first region 417 through which the visual target light flux 407 of the visual target presenting optical system 26 passes. For example, the second region 419 may be formed in the oblique direction with respect to the pupil center position 408 of the subject eye E. For example, in this case, the light source, the index mask, and the relay lens of the alignment index presenting optical system 45 may be arranged in a direction of 45 degrees, a direction of 135 degrees, a direction of 225 degrees, and a direction of 315 degrees on a concentric circle with respect to the optical axis L3. For example, the optical members of the alignment index presenting optical system 45 may be arranged in a combination of the upper, lower, left, and right directions and the oblique direction.

The ophthalmologic device according to the present embodiment may cause the alignment index light flux 409 to be incident on the subject eye E as a convergent light flux. For example, as illustrated in FIGS. 9A to 9C and 13 described above, the alignment index light flux 409 may be incident at a predetermined angle with respect to the optical axis L2 of the visual target light flux 407.

The ophthalmologic device according to the present embodiment may cause the alignment index light flux 409 to be incident on the subject eye E as a parallel light flux. For example, as illustrated in FIGS. 11 and 12 described above, the alignment index light flux 409 may be incident in parallel to the optical axis L2 of the visual target light flux 407. For example, in FIGS. 9A to 9C and 13 described above, the optical members may be provided such that the alignment index light flux 409 is parallel to the optical axis L2 via the composite lens 430.

The ophthalmologic device according to the present embodiment may focus the visual target light flux 407 of the visual target presenting optical system 26 and the alignment index light flux 409 of the alignment index presenting optical system 45 on the fundus of the subject eye E. For example, in this case, the subject can clearly visually recognize both the Landolt ring visual target image and the alignment index image. The ophthalmologic device according to the present embodiment may focus the visual target light flux 407 of the visual target presenting optical system 26 on the fundus of the subject eye E and focus the alignment index light flux 409 of the alignment index presenting optical system 45 in front of the fundus of the subject eye E. For example, in this case, the subject can clearly visually recognize the Landolt ring visual target image, and can visually recognize the alignment index image in a slightly blurred state.

As described above, in the configuration in which the alignment index light flux 409 is condensed in front of the fundus of the subject eye E, the alignment index image is visually recognized by the subject eye E in a fogging state. Therefore, the chance that the alignment index image becomes an accommodation stimulus for the subject eye E can be reduced, and the subjective examination of the subject eye E can be more accurately executed. For example, in a case in which the display 31 is moved when calibrating the spherical power of the subject eye E, the distance between the condensing position of the visual target light flux 407 and the condensing position of the alignment index light flux 409 in the optical axis direction may be maintained in a constant relation by moving at least a part of the optical members of the alignment index presenting optical system 45 together with the display 31 in the optical axis direction. As an example, the condensing position of the alignment index light flux 409 may always be formed on the front side by a distance corresponding to 1.0 D with respect to the condensing position of the visual target light flux 407.

The ophthalmologic device according to the present embodiment may execute an application (a self-optometry application) for automatically progressing the optometry based on an answer input by the subject. For example, in this case, the self-optometry application may store a program for causing the subject to check the presence or absence of alignment deviation. As an example, control for checking alignment deviation may be executed using contact between the face of the subject and at least one of the forehead rest 4 and the jaw support 5 as a trigger signal. As an example, the control for checking the alignment deviation may be periodically executed using a change timing (for example, a timing of transition from the objective measurement to the subjective measurement) of the optometry item for the subject eye E, a timing every time a predetermined time elapses, or the like as a trigger signal. For example, the control unit 70 may execute sound output using a speaker or the like based on such a trigger signal.

### REFERENCE SIGNS LIST

2 housing
6 controller
7 measurement unit
10 objective measurement optical system
25 subjective measurement optical system
26 visual target presenting optical system
30 light projecting optical system
45 alignment index presenting optical system
50 observation optical system
60 correction optical system
70 control unit
75 memory
90 imaging unit
100 ophthalmologic device

## Claims

1. An ophthalmologic device comprising:
a visual target presenting optical system configured to project a visual target light flux toward a subject eye to present a visual target to the subject eye; and
an alignment index presenting optical system configured to project an alignment index toward a fundus of the subject eye to present an alignment index to the subject eye and to present an alignment index light flux to be observed by a subject,
wherein the visual target light flux of the visual target presenting optical system passes through a first region on a pupil plane of the subject eye, and the alignment index light flux of the alignment index presenting optical system passes through a second region on the pupil plane of the subject eye, the second region being different from the first region, and
wherein the second region includes at least a region outside the first region.

2. The ophthalmologic device according to claim 1,
wherein the first region on the pupil plane of the subject eye is a pupil center region including a pupil center of the subject eye, and
wherein the second region on the pupil plane of the subject eye includes at least a region outside the pupil center region.

3. The ophthalmologic device according to claim 1 or 2,
wherein the second region on the pupil plane of the subject eye is located in regions in each of a left direction and a right direction with respect to a pupil center position of the subject eye.

4. The ophthalmologic device according to any one of claims 1 to 3,
wherein the second region on the pupil plane of the subject eye is located in regions in each of an upper direction and a lower direction with respect to a pupil center position of the subject eye.

5. The ophthalmologic device according to any one of claims 1 to 4,
wherein the alignment index presenting optical system is configured to present, as the alignment index, a guide index indicating a direction in which the subject moves the subject eye.
